# EUROPÄISCHE PATENTSCHRIFT

(11) **EP 1 495 036 B1**
(45) Veröffentlichungstag und Bekanntmachung des Hinweises auf die Patenterteilung: **19.08.2009**
(21) Anmeldenummer: 03718689.7
(22) Anmeldetag: 17.03.2003
(51) Int. Cl.: C07F 9/30, C07F 9/38, C07C 17/35, C07C 19/08, C07C 211/62, C07F 9/54, C07D 233/58

(54) **VERFAHREN ZUR HERSTELLUNG VON MONOHYDRO-PERFLUORALKANEN, BIS(PERFLUORALKYL)PHOSPHINATEN UND PERFLUORALKYLPHOSPHONATEN**
METHOD FOR THE PRODUCTION OF MONOHYDRO-PERFLUOROALKANES, BIS(PERFLUOROALKYL)PHOSPHINATES, AND PERFLUOROALKYLPHOSPHONATES
PROCEDE POUR PRODUIRE DES MONOHYDRO-PERFLUOROALCANES, DES BIS(PERFLUOROALKYLE)PHOSPHINATES ET DES PERFLUORALKYLE PHOSPHONATES

(30) Priorität: 16.04.2002 DE 10216995; 08.05.2002 DE 10220547
(43) Veröffentlichungstag der Anmeldung: 12.01.2005
(73) Patentinhaber: Merck Patent GmbH, 64293 Darmstadt (DE)
(72) Erfinder: IGNATYEV, Nikolai, 47058 Duisburg (DE); WEIDEN, Michael, 64285 Darmstadt (DE); WELZ-BIERMANN, Urs, 64646 Heppenheim (DE); HEIDER, Udo, Winchester S022 4HZ (GB); SARTORI, Peter, 86919 Utting (DE); KUCHERYNA, Andriy, 47053 Duisburg (DE); WILLNER, Helge, 45481 Muelheim/Ruhr (DE)
(86) Internationale Anmeldenummer: PCT/EP2003/002744
(87) Internationale Veröffentlichungsnummer: WO 2003/087111

(56) Entgegenhaltungen:
- KOVALEVA T.V. ET AL.: "Perfluoroalkyl- phosphonic acids and their derivatives " JOURNAL OF GENERAL CHEMISTRY USSR., Bd. 59, Nr. 11, 20. April 1990 (1990-04-20), Seiten 2245-2248, XP002246155 CONSULTANTS BUREAU. NEW YORK., US
- PAVLENKO N.V. ET AL.: "Esters of bis(perfluoroalkyl)phosphinic acids" JOURNAL OF GENERAL CHEMISTRY USSR., Bd. 59, Nr. 3, - 20. August 1989 (1989-08-20) Seiten 474-476, XP002246156 CONSULTANTS BUREAU. NEW YORK., US
- MAHMOOD T ET AL: "New perfluoroalkyl- phosphonic and bis(perfluoroalkyl) phosphinic acids and their precursors" INORGANIC CHEMISTRY, AMERICAN CHEMICAL SOCIETY, EASTON, US, Bd. 25, Nr. 18, 1986, Seiten 3128-3131, XP002221561 ISSN: 0020-1669
- GOSLING, K. ET AL: "Preparation and hydrolysis of tertiary alkyl (perfluoroalkyl)phosphines" JOURNAL OF THE CHEMICAL SOCIETY [SECTION] A: INORGANIC, PHYSICAL, THEORETICAL (1968), (8), 1909-14 , 1968, XP002246157
- HASZELDINE R.N.: "Reactions of fluorocarbon radicals. Part XII." JOURNAL OF THE CHEMICAL SOCIETY., 1953, Seiten 3761-3768, XP002246158 CHEMICAL SOCIETY. LETCHWORTH., GB ISSN: 0368-1769
- BERGMAN E.: "Decarbethoxylation of perfluoroacid esters" JOURNAL OF ORGANIC CHEMISTRY., Bd. 23, Nr. 3, 1958, Seiten 476-477, XP002246159 AMERICAN CHEMICAL SOCIETY. EASTON., US ISSN: 0022-3263 in der Anmeldung erwähnt

## Beschreibung

Die vorliegende Erfindung betrifft ein Verfahren zur Herstellung von Monohydro-Perfluoralkanen, Bis(perfluoralkyl)phosphinaten und Perfluoralkylphosphonaten das zumindest die Behandlung wenigstens eines Perfluoralkylphosphorans mit wenigstens einer Base in einem geeigneten Reaktionsmedium umfaßt.

Monohydro-Perfluoralkane sind seit langem bekannt und haben breite Anwendung in verschiedenen Bereichen gefunden, u.a. als ozonfreundliche Kühlmittel (WO 01/40400, WO 01/23494, WO01/23491, WO99/36485, WO 98/08913), als Reinigungsmittel (WO01/32323), als Bestandteil von Ätzmitteln für den Bereich der Mikroelektronik (US2001/0005637, US 6228775) in Feuerlöschern (WO010/5468, Combust. Flame, 121, Nr. 3 (2000) Seiten 471-487, CN 1218702), als Blähmittel in Schäumen (US6225365, WO01/18098) sowie zur Herstellung polymerer Materialien und potentieller Anästhetika (Anesth. Analg (N.Y.), 79, Nr. 2 (1994), Seiten 245-251, T. Hudlicky et al., J. of Fluorine Chem., 59, Nr. 1 (1992), Seiten 9-14).

Einige dieser Monohydro-Perfluoralkane, wie z.B. Pentafluorethan, werden bereits großtechnisch im Tonnenmaßstab produziert, wobei die Herstellung üblicherweise durch katalytische Hydrofluorierung von chlorierten Kohlenwasserstoffen erfolgt (WO01/77048, EP 1052235).
Nachteilig bei diesen Verfahren ist einerseits die Gefahr, die mit dem Einsatz von Fluorwasserstoff bei relativ hohen Temperaturen verbunden ist. Des weiteren sind besondere Katalysatoren erforderlich, die zuvor durch vergleichsweise aufwendige Verfahren hergestellt werden müssen. Ein weiterer Nachteil dieser Verfahren besteht darin, daß die Herstellung der chlorierten Kohlenwasserstoffe unter Verwendung von Chlor in ökologischer Hinsicht bedenklich ist und die Produktionskosten weiter erhöht. Schließlich eignen sich die bekannten Verfahren zur Herstellung von Pentafluorethan nicht ohne weiteres zur Herstellung von längerkettigen Monohydro-Perfluoralkanen, wie z.B. 1-Hydro-nonafluorbutanen.

Des weiteren sind einige weitere Verfahren bekannt, gemäß denen die Herstellung von Pentafluorethan unter Verwendung spezieller Fluorierungsmittel, wie beispielsweise BrF₃ (R. A. Devis, J. Org. Chem. 32 (1967), Seite 3478), XeF₂ (JP2000/119201), SF₄ (G. Siegemund, Liebigs Ann. Chem., 1979, Seite 1280, E.R. Bissell, J. of Organic Chem., 29, (1964), Seite 1591), SbF₅ (G.G. Belenkii et al., Izv. Akad. Nauk SSSR, Ser.. Khim., 1972, Seiten 983, Chem. Abstr. 77 (1972) 75296, A.F.Ermolov et al., Zh. Org. Khim., 17 (1981), Seite 2239, J. Org. Chem. USSR (Engl. Translation), 17 (1981), Seite 1999, US 2426172), MoF₆ (L.D. Shustov et al., Zh. Obshch. Khim., 53 (1983), Seite 103, J. Gen. Chem. USSR (Engl. Translation), 53 (1983), Seite 85) und CoF₃ (US6162955), gelingt.

Die vorstehend genannten Verfahren haben jedoch keine industrielle Bedeutung erlangt, da sowohl die jeweiligen Ausgangsverbindungen als auch die Fluorierungsmittel selbst sehr teuer sind.

Zur Herstellung langkettiger Monohydro-Perfluoralkane sind dagegen nur wenige Verfahren bekannt.

Gemäß einem ersten Verfahren werden die Monohydro-Perfluoralkane durch Decarboxylierung von Salzen perfluorierter Carboxylsäuren (J.D. LaZerte et al., J. Am. Chem. Soc., 75 (1953), Seite 4525; R.N. Haszeldine, J. Chem. Soc. 1953, Seite 1548) oder entsprechender Ester (E. Bergman, J. Org. Chem., 23, (1958) Seite 476) durch die Behandlung mit starken Basen, wie beispielsweise Natriumethylat, hergestellt. Nach einem anderen Verfahren erfolgt die Herstellung der Monohydro-Perfluoralkane durch die Behandlung von perfluorierten Ketonen, die am Carbonylkohlenstoffatom eine Trifluormethyl-Gruppe aufweisen, mit wäßrigem Alkali (L.V. Saloutina et al., Izv. Akad. Nauk SSSR, Ser. Khim., 1984, Nr. 5, Seiten 1114-1116, Chem. Abstr. 101 (1984) 210504x). Nachteilig ist auch bei diesen Verfahren der Einsatz teuerer Ausgangsmaterialien sowie die erforderlichen hohen Temperaturen.

Die Herstellung von 1-Hydro-n-nonafluorbutan gelingt ferner durch die Reduktion von Perfluorbutyliodid mit verschiedenen Reduktionsmitteln, wie z.B. Zinkstaub in Methanol (T. Hudlicky et al., J. of Fluorine Chem., 59, Nr. 1 (1992), Seiten 9-14), Natriummethoxid (J.L. Howell et al., J. of Fluorine Chem., 72, Nr. 1 (1995), Seiten 61-68), durch Wasserstoff in der Gasphase bei hohen Temperaturen (EP 6 32 Q01), sowie mit Hilfe des Thalliumkomplexes [TaC_{P2}(C₂H₄)H] (P.H. Russel et al., Polyhedron 17, Nr. 7 (1998), Seiten 1037-1043).

Diese Verfahren haben jedoch ebenfalls den Nachteil, daß sie von der Ausgangsverbindung Perfluorbutyljodid ausgehen, die nur durch vergleichsweise teure Produktionsverfahren herzustellen ist.

Die Aufgabe der vorliegenden Erfindung bestand daher darin, ein Verfahren zur Verfügung zu stellen, welches die einfache und kostengünstige Herstellung von Monohydro-Porfluoralkanen in guten Ausbeuten ermöglicht. Vorzugsweise sollen die Monohydro-Penfluoralkane in hoher Reinheit erhalten werden. Eine weitere Aufgabe bestand in der Bereitstellung von Bis(perfluoralkyl)phosphinaten und Perfluoralkylphosphonaten.

Diese Aufgabe wurde durch das erfindungsgemäße Verfahren zur Herstellung von Monohydro-Perfluoralkanen, bevorzugt solchen der allgemeinen Formel CₙHF₂ₙ₊₁ worin 1 ≤ n ≤ 8, vorzugsweise 1≤,n≤ 4, bedeuten, Bis(perfluoralkyl)phosphinaten und Perfluoralkylphosphonaten gelöst, welches zumindest die Behandlung wenigstens eines Perfluoralkylphosphorans mit wenigstens einem Erdalkalimetallhydroxid, einer metallorganischen Verbindung oder einer organischen Base und gegebenenfalls einer Säure in Wasser und/oder einem oder mehreren organischen Lösungsmitteln als Reaktionsmedium umfaßt.

Erfindungsgemäß können zur Herstellung von Monohydro-Perfluoralkanen nach dem erfindungsgemäßen Verfahren jeweils ein Perfluoralkylphosphoran oder Gemische aus zwei oder mehreren Perfluoralkylphosphoranen zum Einsatz kommen. Vorzugsweise wird jeweils nur ein Perfluoralkylphosphoran nach dem erfindungsgemäßen Verfahren umgesetzt.

Die Herstellung der in dem erfindungsgemäßen Verfahren zum Einsatz kommenden Perfluoralkylphosphorane kann nach üblichen, dem Fachmann bekannten Methoden erfolgen.

Vorzugsweise werden die Perfluoralkylphosphorane durch elektrochemische Fluorierung geeigneter Ausgangsverbindungen hergestellt, wie in V. Ya. Semenii et al., Zh. Obshch.Khim., 55, Nr. 12 (1985), Seiten 2716-2720; N. Ignatiev, J. of Fluorine Chem., 103 (2000), Seiten 57-61 sowie der WO 00/21969 beschrieben. Die entsprechenden Beschreibungen werden hiermit als Referenz eingeführt und gelten als Teil der Offenbarung.

In einer bevorzugten Ausführungsform des erfindungsgemäßen Verfahrens kommt wenigstens ein Perfluoralkylphosphoran der allgemeinen Formel I

(CₙF₂ₙ₊₁)ₘPF₅₋ₘ I

worin 1 ≤ n ≤ 8, vorzugsweise 1 ≤ n ≤ 4 und m jeweils 1, 2 oder 3 bedeutet, zum Einsatz.

Besonders bevorzugte Perfluoralkylphosphoranverbindungen sind ausgewählt aus der Gruppe bestehend aus Difluortris(pentafluorethyl)phosphoran, Difluortris(n-nonafluorbutyl)phosphoran, Difluortris(n-heptafluorpropyl)-phosphoran und Trifluorbis(n-nönafluorbutyl)phosphoran.

Vorzugsweise erfolgt die Behandlung der Perfluoralkylphosphoranverbindung(en) nach dem erfindungsgemäßen Verfahren jeweils unter Verwendung nur einer Base. Selbstverständlich kommt aber auch der Einsatz von Gemischen aus zwei oder mehr Basen in dem erfindungsgemäßen Verfahren in Betracht. Die jeweiligen Basen können auch in Form entsprechender Solvate, vorzugsweise in Form entsprechender Hydrate, oder in Form von üblichen, dem Fachmann bekannten Anlagerungsverbindungen zum Einsatz kommen.

Sofern ein Erdalkalimetallhydroxid als Base in dem erfindungsgemäßen Verfahren zum Einsatz kommt, kann dieses vorzugsweise ausgewählt werden aus der Gruppe bestehend aus Bariumhydroxid, Bariumhydroxidoctahydrat und Calciumhydroxid.

Ebenfalls bevorzugt kann eine organische Base oder metallorganische Verbindungen in dem erfindungsgemäßen Verfahren zur Herstellung von Monohydro-Perfluoralkanen zum Einsatz kommen. Die organische Base kann bevorzugt ausgewählt werden aus der Gruppe von Alkylammoniumhydroxiden, Arylammoniumhydroxiden, Alkylarylammoniumhydroxiden, Alkylphosphoniumhydroxiden, Arylphosphoniumhydroxiden, Alkylarylphosphoniumhydroxiden, Alkylaminen, Arylaminen, Alkylarylaminen, Alkylphosphinen, Arylphosphinen und Alkylarylphosphinen.

Bevorzugte metallorganische Verbindungen können ausgewählt werden aus der Gruppe bestehend aus Metallalkyloxiden, vorzugsweise Alkalimetallalkoxiden, Metallaryloxiden, Metallalkylthiooxiden, Metallarylthiooxiden, Alkylmetallen, Arylmetallen und Grignard-Reagenzien,

Sofern eine der vorstehend genannten Klassen von Basen einen Alkyl-Rest aufweist, kann dieser bevorzugt 1 bis 4 Kohlenstoffatome enthalten. Weist die entsprechende Base zwei oder mehr Alkylreste auf, können diese jeweils gleich oder verschieden sein, wobei gleiche Alkylreste bevorzugt sind.

Weist eine der vorstehend genannten Klassen von Basen einen Aryl-Rest auf, kann dieser bevorzugt ein unsubstituierter oder wenigstens einfach substituierter Phenyl-Rest sein.

Das Reaktionsmedium des erfindungsgemäßen Verfahrens kann Wasser sein, gegebenenfalls im Gemisch mit einem oder mehreren organischen Lösungsmitteln, wobei erfindungsgemäß auch zweiphasige Systeme, wie zum Beispiel Gemische aus Wasser und Kohlenwasserstoff, umfaßt werden.

Ebenfalls können in dem erfindungsgemäßen Verfahren zur Herstellung von Monohydro-Perfluoralkanen ein oder mehrere organische Lösungsmittel zum Einsatz kommen, wobei für den Fall, daß wenigstens zwei Lösungsmittel zum Einsatz kommen, diese ggf. in Form eines zweiphasigen Systems vorliegen können.

Geeignete organische Lösungsmittel, die jeweils allein oder in beliebiger Kombination untereinander, ggf. auch im Gemisch mit Wasser in dem erfindungsgemäßen Verfahren zum Einsatz kommen, können bevorzugt ausgewählt werden aus der Gruppe bestehend aus Alkoholen, Ethern, Acylamiden, Sulfoxiden, Sulfonen, Nitrilen und Kohlenwasserstoffen.

Bevorzugte Alkohole sind solche, mit 1 bis 4 Kohlenstoffen im Alkylteil. Vorzugsweise können entsprechende Alkohole ausgewählt werden aus der Gruppe bestehend aus Methanol, Ethanol, isopropanol und Mischungen aus wenigstens zwei dieser vorstehend genannten Alkohole.

Die Menge des aus dem/den jeweils eingesetzten Perfluoralkylphosphoran(en) gebildeten Monohydro-Perfiuoralkans sowie die Art der weiteren Reaktionsprodukte kann nach dem erfindungsgemäßen Verfahren gezielt gesteuert werden, beispielsweise Durch die Wahl der Parameter können z. B. gezielt ein, zwei oder drei Perfluoralkylgruppen aus dem jeweils eingesetzten Difluortrisperfluoralkylphosphoran abgespalten werden.

Bei der Abspaltung einer Perfluoralkylgruppe aus dem jeweiligen Difluortrisperfluoralkylphosphoran wird neben dem gewünschten . Monohydro-Perfluoralkan u.a. auch das entsprechende Bis(perfluoralkyl)phosphinat gebildet.

Bei der Abspaltung von zwei Perfluoralkylgruppen aus dem jeweiligen Difluortrisperfluoralkylphosphoran wird neben dem gewünschten Monohydro-Perfluoralkan u.a. auch das entsprechende Perfluoralkylphosphonat gebildet.

Werden alle drei Perfluoralkylgruppen aus dem jeweiligen Difluortrisperfluoralkylphosphoran abgespalten, wird neben dem gewünschten Monohydro-Perfluoralkan u.a. auch das entsprechende Phosphat erhalten.

Die jeweils für die gewünschte Kombination aus dem entsprechenden Monohydro-Perfluoralkan, dessen Menge und den jeweiligen weiteren Reaktionsprodukten optimale Wahl der Parameter kann vom Fachmann anhand einfacher Vorversuche ermittelt werden.

Sofern z.B. die Abspaltung einer Perfluoralkylgruppe aus dem jeweils eingesetzten Difluortrisperfluoralkylphosphoran erfolgen soll, kann das erfindungsgemäße Verfahren bevorzugt bei einer Temperatur von -10 °C bis 100 °C und einem moläquivalenten Verhältnis von Difluortrisperfluoralkylphosphoran zu Base von 1:3 durchgeführt werden.

Soll z.B. die Abspaltung von zwei Perfluoralkylgruppen aus dem jeweils eingesetzten Difluortrisperfluoralkylphosphoran erfolgen, kann das erfindungsgemäße Verfahren bevorzugt bei einer Temperatur von 50 °C bis 150 °C und einem moläquivalenten Verhältnis von Difluortrisperfluoralkylphosphoran zu Base von 1:4 durchgeführt werden.

Sofern beispielsweise die Abspaltung der drei Perfluoralkylgruppen aus dem jeweils eingesetzten Difluortrisperfluoralkylphosphoran erfolgen soll, kann das erfindungsgemäße Verfahren bevorzugt bei einer Temperatur von 100 °C bis 250 °C und einem moläquivalenten Verhältnis von Difluortrisperfluoralkylphosphoran zu Base von 1:5 durchgeführt werden.

Die nach dem erfindungsgemäßen Verfahren hergestellten Monohydro-Perfluoralkane können, sofern erforderlich, nach üblichen, dem Fachmann bekannten Verfahren isoliert und ggf. gereinigt werden.
Sofern es sich um leicht flüchtige Verbindungen handelt, können diese beispielsweise über Kondensation in einer oder mehreren Kühlfallen, die vorzugsweise mit flüssigem Stickstoff oder Trockeneis gekühlt werden, aus dem Reaktionsgemisch isoliert werden.

Gegebenenfalls erfolgt die Isolierung und ggf. Reinigung weiterer Reaktionsprodukte ebenfalls nach üblichen, dem Fachmann bekannten Verfahren, wie beispielsweise durch fraktionierte Kristallisation oder Extraktion mit geeigneten Lösungsmitteln.

Wird das Perfluoralkylphosphoran mit einer anorganischen Base (b) umgesetzt, so können die damit gebildeten Bis(perfluoralkyl)phosphinate und Perfluoralkylphosphonate direkt oder nach Isolierung mit einer Säure, bevorzugt mit Schwefelsäure, in die entsprechenden Bis(perfluoralkyl)phosphinsäuren und Perfluoralkylphosphonsäuren überführt werden.

Die so erhaltenen Bis(perfluoralkyl)phosphinsäuren und Perfluoralkylphosphonsäuren können durch Neutralisation, vorzugsweise mit organischen Basen (c) in die Salze überführt werden.

Durch Auswahl geeigneter Basen werden vorzugsweise der teil- und peralkylierten Ammonium-, Phosphonium-, Sulfonium-, Pyridinium-, Pyridazinium-, Pyrimidinium-, Pyrazinium-, lmidazolium-, Pyrazolium-, Thiazolium-, Oxazolium- und Triazoliumsalze-salze dargestellt.

Besonders bevorzugt werden Salze mit einem Kation ausgewählt aus der Gruppe 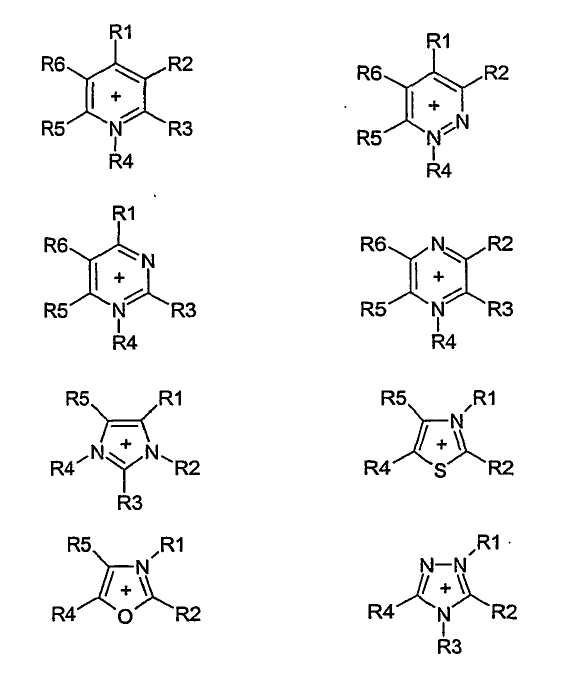 wobei R¹ bis R⁵ gleich oder verschieden, gegebenenfalls durch eine Einfach- oder Doppelbindung direkt miteinander verbunden sind und jeweils einzeln oder gemeinsam folgende Bedeutung haben:
- H,
   - Halogen, wobei die Halogene nicht direkt mit N verbunden werden,
   - Alkylrest (C₁ bis C₈) der teilweise oder vollständig durch weitere Gruppen, vorzugsweise F, Cl, N(CₙF₍₂ₙ₊₁₋ₓ₎Hₓ)₂, O(CₙF₍₂ₙ₊₁₋ₓ₎Hₓ), SO₂(CₙF₍₂ₙ₊₁₋ₓ₎Hₓ), CₙF(₂ₙ₊₁₋ₓ)Hₓ mit 1 <n<6 und 0<x≤2n+1 substituiert sein kann, dargestellt.

Diese Salze können auch gewonnen werden, wenn das nach der Umsetzung des Perfluoralkylphosphorans mit einer anorganischen Base (b) gebildete Salz direkt oder nach Isolierung umgesalzt wird.

Die Umsalzungen können mit Aryl-, Alkyl- oder Alkylarylammonium- oder - phoshoniumsalzen erfolgen. Bevorzugt werden Hexafluorophosphate, Tetrafluoroborate, Hexafluoroarsenate, Sulfate, Fluoride, Chloride oder Bromide verwendet.

Die so erhaltenen Salze können in üblicher, dem Fachmann bekannter Weise, aufgearbeitet werden.

Das erfindungsgemäße Verfahren zur Herstellung von Monohydro-Perfluoralkanen ermöglicht die einfache, kostengünstige und sichere Herstellung dieser Verbindungen in sehr guten Ausbeuten. Insbesondere können die als Ausgangsverbindungen zum Einsatz kommenden Perfluoralkylphosphorane in großen Mengen preiswert hergestellt werden.

Vorteilhaft ist ferner, daß die gemäß dem erfindungsgemäßen Verfahren erhaltenen Nebenprodukte, wie beispielsweise die Bis(perfluoralkyl)phosphinate und die Perfluoralkylphosphonate selbst wertvolle Rohstoffe darstellen, die sich u.a. zur Herstellung der entsprechenden Bis(perfluoralkyl)phosphinsäuren und Perfluoralkylphosphonsäuren eignen und somit einer wirtschaftlichen Nutzung zugeführt werden können. Durch die Neutralisation mit geeigneten Basen können daraus z.B. Bis(perfluoralkyl)phosphinate und Perfluoralkylphosphonate hergestellt werden, die sich zur Verwendung als ionische Flüssigkeiten, Tenside oder Phasen-Transfer-Katalysatoren eignen.

Das hat weiterhin den Vorteil, daß die Umweltbelastung bei der Umsetzung nach dem erfindungsgemäßen Verfahren gering gehalten wird, was sich ferner positiv auf die Produktionskosten der nach dem erfindungsgemäßen Verfahren hergestellten Monohydro-Perfluoralkane auswirkt.

Die jeweiligen Monohydro-Perfluoralkane werden ferner unmittelbar nach ihrer Herstellung, d.h. ohne aufwendige Reinigungsschritte, in hoher Reinheit erhalten.

Im folgenden wird die Erfindung anhand von Beispielen erläutert. Diese Beispiele dienen lediglich der Erläuterung der Erfindung und schränken den allgemeinen Erfindungsgedanken nicht ein.

### Beispiel 1:

Innerhalb von einer Stunde werden 13,46 g (31,6 mmol) Difluortris(pentafluorethyl)phosphoran unter Rühren über einen Tropftrichter zu 96,5 g (131,1 mmol) einer 20 Gew.%-igen, wässrigen Lösung von Tetraethylammoniumhydroxid bei Raumtemperatur gegeben. Hierbei wird ein Erwärmen der Reaktionsmischung beobachtet. Anschließend wird das Reaktionsgemisch für 30 Minuten auf 80 °C erhitze. Das durch die Hydrolyse des Difluortris(pentafluorethyl)phosphorans gebildete, gasförmige Pentafluorethan wird in einer nachgeschalteten Falle aufgefangen, die mit flüssigem Stickstoff gekühlt wird.
In der gekühlten Falle werden 7,49 g festes Pentafluorethan erhalten. Die Ausbeute an Pentafluorethan beträgt 98,8 %, bezogen auf die zwei abgespalteten Pentafluorethylgruppen.

Die für Pentafluorethan ermittelten chemischen Verschiebungen entsprechen den in Beispiel 1 angegebenen Werten.

Die im Kolben verbliebene Lösung wird am Rotationsverdampfer eingeengt und der so erhaltene Feststoff unter vermindertem Druck bei 120 Pa und einer Temperatur von 100 °C getrocknet. Es werden 24,67 g weißes kristallines [(C₂H₅)₄N]₂[C₂F₅PO₃]·2 [(C₂H₅)₄N]F.8H₂O

Das [(C₂H₅)₄N]₂[C₂F₅PO₃]-2 [(C₂H₅)₄N]F.8H₂O wird mittels ¹H-, ¹⁹F- und ¹⁹F ³¹p-NMR-Spektroskopie sowie durch Elementaranalyse charakterisiert:

Die ¹⁹F-, ¹H- und ³¹p-NMR-Spektren werden auf einem Bruker Avance 300 Spektrometer bei einer Frequenz von 282.4 MHz für ¹⁹F und 121,5 MHz für ³¹ P aufgenommen.

¹⁹F-NMR-Spektrum:
(Lösungsmittel Acetonitril-D₃, Referenz CCl₃F, δ, ppm)
   -79,41 dt (CF₃); -126,74 dq (CF₂); -111,74 (2F-); ²Jp,_{F} = 54,0 Hz; ³J_{P,F} = 1,1 H_{z}; ³J_{F,F} = 1,0 Hz

¹H-NMR-Spektrum:
(Lösungsmittel Acetonitril-D₃, Referenz TMS, δ, ppm)
   1,21 tm (CH₃); 3,28 q (CH₂); ³j_{H,H} = 7,3 Hz

Es findet ein Protonenaustausch zwischen den H₂O-Molekülen und dem Deuterium des Lösungsmittels statt;
³¹ P-NMR-Spektrum:
(Lösungsmittel Acetonitril-D₃, Referenz 85 Gew.-%ige H₃PO₄ - 15 % D₂O in
Acetonitril-D₃, δ, ppm)
-1,77 t; ²J_{P,F} = 54,2 Hz

Elementaranalyse:
berechnet für C₃₄H₉₆F₅N₄O₁₁P: C: 47,31 %; H: 11,21 %; N: 6,49 % gefunden: C: 47,37 %; H: 10,80 %; N: 6,40 %

### Beispiel 2 :

In einem Kolben werden 50,38 g (159,7 mmol) Bariumhydroxid-octahydrat in 100 cm³ Wasser suspendiert und die so erhaltene Suspension bei einer Badtemperatur von 65.-70 °C erwärmt. Anschließend werden innerhalb von 30 Minuten 22,68 g (53,2 mmol) Difluortris(pentafluorethyl)phosphoran über einen Tropftrichter unter Rühren zugegeben. Anschließend wird das Reaktionsgemisch auf eine Temperatur von 150 °C erwärmt und für zwei Stunden bei dieser Temperatur gerührt.
Das durch die Hydrolyse des Difluortris(pentafluorethyl)phosphorans gebildete, gasförmige Pentafluorethan wird in einer nachgeschalteten Falle aufgefangen, die mit Trockeneis gekühlt wird.
In der gekühlten Falle werden 10,00 g flüssiges Pentafluorethan erhalten. Die Ausbeute an Pentafluorethan beträgt 78,3 %, bezogen auf die zwei Pentafluorethylgruppen, die unter diesen Bedingungen aus dem Difluortris(pentafluorethyl)phosphoran abgespalten werden.
Der im Kolben verbliebene Rückstand wird in 50 cm³ Wasser aufgenommen und mit einer wäßrigen Fluorwasserstoff Lösung neutralisiert. Der gebildete Niederschlag von Bariumfluorid wird abfiltriert. Zur Isolierung des Bariumpentafluorethylphosphonats wird das Wasser unter vermindertem Druck entfernt. Der so erhaltene weiße Feststoff wird unter vermindertem Druck bei 120 Pa und einer Badtemperatur von 100 °C für eine Stunde getrocknet. Es werden 10,6 g Bariumpentafluorphosphonat ([C₂F₅P(O)O₂]Ba) erhalten, welches noch ca. 2 Gew.-% Bariumfluorid enthält, entsprechend einer Ausbeute von 59,2 %, bezogen auf das eingesetzte Difluortris(pentafluorethyl)phosphoran.

Das Pentafluorethan wird mittels ¹H-, ¹⁹F-, das Bariumpentafluorphosphonat mittels ¹⁹F- und ³¹P-NMR-Spektroskopie charakterisiert.

Die für Pentafluorethan ermittelten chemischen Verschiebungen entsprechen den in Beispiel 1 angegebenen Werten.

Bariumpentafluorethylphosphonat

Die ¹⁹F-, ¹H- und ³¹p-NMR-5pektren werden auf einem Bruker Avance 300 Spektrometer bei einer Frequenz von 282.4 MHz für ¹⁹F und 121,5 Hz für ³¹P aufgenommen.
¹⁹F-NMR-Spektrum:
(Lösungsmittel D₂O, Referenz CF₃COOH in D₂O = 76,53 ppm, δ, ppm)
-81,99 td (CF₃); -126,25 dq (CF₂);
²J_{P,F} = 70,5 Hz; ³J_{FF} =1.8 Hz; ³J_{P,F} = 0,5 Hz
³¹ P-NMR-Spektrum:
(Lösungsmittel D₂O, Referenz 85 Gew.%ige H₃PO₄ in D₂O, δ, ppm)
2,88 t; ²Jp,_{F} = 70,3 Hz

### Beispiel 3:

In einem Kolben werden 16,70 g (52,9 mmol) Bariumhydroxid-octahydrat in 20 cm³ Wasser suspendiert und die so erhaltene Suspension bei einer Badtemperatur von 70-80 °C erwärmt. Anschließend werden mit Hilfe eines Tropftrichters innerhalb von 30 Minuten 17,79 g (24,5 mmol) Difluortris(n-nonafluorbutyl)phosphoran unter Rühren zugegeben. Anschließend wird das Reaktionsgemisch bei einer Badtemperatur von 120 °C erwärmt und für eine Stunde bei dieser Temperatur gerührt.
Das durch die Hydrolyse des Difluortris(n-nonafluorbutyl)phosphorans gebildete, gasförmige 1 H-n-Nonafluorbutan wird in einer nachgeschalteten Falle aufgefangen, die mit flüssigem Stickstoff gekühlt wird.
In der gekühlten Falle werden 7,72 g festes 1 H-n-Nonafluorbutan erhalten. Die Ausbeute an 1 H-n-Nonafluorbutan beträgt 71,6 %, bezogen auf die zwei n-Nonafluorbutylgruppen, die unter diesen Bedingungen aus dem Difluortris(n-nonafluorbutyl)phosphoran abgespalten werden.

Der im Kolben verbliebene Rückstand wird in 50 cm³ Wasser aufgenommen und mit einer wäßrigen Fluorwasserstoff Lösung neutralisiert. Der gebildete Niederschlag von Bariumfluorid wird abfiltriert.

Zur Isolierung des Barium-n-nonafluorbutylphosphonats wird das Wasser unter vermindertem Druck entfernt. Der so erhaltene weiße Feststoff wird unter vermindertem Druck bei 120 Pa und einer Badtemperatur von 100 °C für eine Stunde getrocknet. Es werden 7,0 g Barium-n-nonafluorbutylphosphonat ([n-C₄F₉P(O)O₂]Ba) erhalten, welches noch ca. 2 Gew.-% Bariumfluorid enthält, entsprechend einer Ausbeute von 64,87 %, bezogen auf das eingesetzte Difluortris(pentafluorethyl)phosphoran.

Das 1H-n-Nonafluorbutan wird mittels ¹H-, ¹⁹ F-, das Barium-n-nonafluorbutylphosphonat mittels ¹⁹F- und ³¹P-NMR-Spektroskopie charakterisiert,

Die für 1-H-Nonafluorbutan ermittelten chemischen Verschiebungen entsprechen den in Beispiel 3 angegebenen Werten.

Barium-n-nonafluorbutylphosphonat

¹⁹F-NMR-Spektrum:
(Lösungsmittel D₂O, Referenz CF₃COOH in D₂O = 76,53 ppm, δ, ppm)
-81,77 tt (CF₃); -122,29 m (CF₂); -123,66 dtm (CF₂); -126,76 tm (CF₂);
²Jp,F = 75,8 Hz; ⁴J_{F,F} = 9,7 Hz; ⁴J_{F,F} = 13,8 Hz; J_{F},_{F} = 3,6 Hz

³¹ P-NMR-Spektrum:
(Lösungsmittel D₂O, Referenz 85 Gew.-%ige H₃PO₄ in D₂O, δ, ppm) 2,22 t; ²Jp,F = 76,1 Hz

### Beispiel 4:

Zu einer gerührten Lösung von 3,42 g (10,2 mmol) Bariumpentafluoroethylphosphonat in 50 cm³ Wasser werden 1,0 g (10,2 mmol) einer 100 %igen Schwefelsäure H₂SO₄gegeben. Es bildet sich ein Niederschlag von Bariumsulfat, der durch Filtrieren abgetrennt wird. Das so erhaltene Filtrat wird unter vermindertem Druck vollständig eingeengt und bei 125 Pa und einer Temperatur des Ölbades von 100 °C weitere 6 Stunden getrocknet. Es werden 1,75 g einer hochviskosen Flüssigkeit von Pentafluoroethylphosphonsäure C₂F₅(O)(OH)₂ erhalten, entsprechend einer Ausbeute von 83,8 %.
¹⁹F-NMR-Spektrum:
(Lösungsmittel: Acetonitril-D₃, Referenz CCl₃F, δ, ppm)
-81,03 t (CF₃); -126,74 dq (CF₂); J²_{P,F}=89,4 Hz, J³_{F,F}= 1,6 Hz

¹H-NMR-Spektrum:
(Lösungsmittel: Acetonitril-D₃, Referenz TMS, δ, ppm)
11,26 br.s (OH)
³¹P-N MR-Spektrum

(Lösungsmittel: Acetonitril-D₃; Referenz 85 Gew.-% H₃PO₄-15 Gew,-% D₂O in Acetonitril-D₃): -3,40 t, J²_{P,F} = 89,6 Hz.

Diese Daten entsprechen den aus der Literaturveröffentlichung von T. Mahmood und J.M. Shreeve, in Inorg. Chem., 25 (1986), Seiten 3128-3131 bekannten Werten.

### Beispiel 5:

Eine Lösung von 0,492 g (2,46 mmol) gemäß Beispiel 4 hergestellter Pentafluorethylphosphonsäure in 10 cm³ Wasser wird mit 3,015 g 20-Gew.% wässrigen Tetraethylammoniumhydroxid durch langsame Zugabe bei Raumtemperatur unter Rühren neutralisiert. Das Wasser wird unter vermindertem Druck abgedampft und der so erhaltene Rückstand unter vermindertem Druck von 120 Pa und einer Badtemperatur von 50 °C für 2 Stunde getrocknet. Es werden 1.115 g weißer Feststoff von Bis(tetraethylammonium) pentafluoroethylphosphonat erhalten. Die Ausbeute beträgt 99,0 % , bezogen auf die eingesetzte Pentafluorethylphosphonsäure,
Bis(tetraethylammonium)pentafluoroethylphosphonat wurde mittels 19F, ³¹P und ¹H-NMR-Spektroskopie charakterisiert:

¹⁹F NMR spectrum, ppm :
(Lösungsmittel: Acetonitril-D₃ ; Referenz: CCl₃F):
-79,49 s (CF₃) ; -122,10 d (CF₂) J²_{P,F} = 54.6 Hz.

¹H NMR spectrum, ppm:
(Lösungsmittel: Acetonitril-D₃ ; Referenz: TMS):
1,20 tm (12H, 4CH₃); 3,29 q (8H, 4CH₂) J³_{H,H} = 7.3 Hz.
³¹P NMR spectrum, ppm:
(Lösungsmittel: Acetonüril-D₃ ; Referenz: 85 % H₃PO₄):
-2.28 t ; J²_{P,F} =54.9 Hz.

### Beispiel 6:

Eine Lösung aus Nonafluor-n-butylphosphonsäure, hergestellt gemäß Beispiel 4 aus 3,73 g (8,57 mmol) Bariumnonafluor-n-butylphosphonat und 0,839 g 100-Gew. % Schwefelsäure in 20 cm³ Wasser , wird mit 20-Gew.% wässrigem Tetraethylammoniumhydroxid durch langsame Zugabe bei Raumtemperatur unter Rühren neutralisiert (ph = 7). Das Wasser wird unter vermindertem Druck abgedampft und der so erhaltene Rückstand unter vermindertem Druck von 120 Pa und einer Badtemperatur von 60 °C für 2 Stunde getrocknet.
Es werden 4,59 g Feststoff von Bis(tetraethylammonium)-nonafluor-n-butylphosphonat erhalten, Die Ausbeute beträgt 96,0 % , bezogen auf die eingesetzte Bariumnonafluor-n-butylphosphonat.
Bis(tetraethylammonium)nonafluor-n-butylphosphonat wurde mittels ¹⁹F, ³¹P und¹H-NMR-Spektroskopie charakterisiert:

¹⁹F NMR spectrum, ppm :
(Lösungsmittel: Acetonitril-D₃ ; Referenz: CCl₃F):
-80,37 tt (CF₃) ; -119,57 m (CF₂) ; -119,72 dm (CF₂) ; -124,80 m (CF₂) ;
J²_{P.F} = 55.6 Hz ; J³_{F},_{F} =4.3 Hz ; j⁴ _{F},_{F} =9.5 Hz.

¹H NMR spectrum, ppm:
(Lösungsmittel: Acetonitril-D₃ ; Referenz: TMS):
1,23 tm (12H, 4CH₃) ; 3,27 q (8H, 4CH₂) ; J³H,H = 7.4 Hz.

³¹P NMR spectrum, ppm:
(Lösungsmittel: Acetonitril,D₃ ; Referenz: 85 % H₃PO₄):
-2.06 t ; J²_{P,F} =56.5 Hz.

### Beispiel 7

1,43 g der gemäß Beispiel 4 hergestellten Pentafluoroethylphosponsäure werden in 15 cm³ Wasser gelöst und mit 10-Gew.% wässrigem Kaliumhydroxid durch langsame Zugabe bei Raumtemperatur unter Rühren neutralisiert (pH = 7). Zu der so erhaltenen wässrige Lösung von Dikaliumpentafluor-ethylphosponat wird unter ständigem Rühren eine Lösung von 2,09 g (11,9 mmol) 1-Ethy1-3-methylimidazoliumchlortde in 3 cm³ Wasser bei Raumtemperatur gegeben. Das Wasser wird unter vermindertem Druck abgedampft und der so erhaltene Rückstand unter : vermindertem Druck von 120 Pa und einer Badtemperatur von 60 °C für 1 Stunde getrocknet. Anschließend werden 10 cm³ Isopropylalkohol zum Rückstand gegeben, ein weißer Niederschlag abfiltriert und zwei Mal mit 5 cm³Isopropylalkohol gewaschen. Der Isopropylalkoho! wird unter vermindertem Druck abgedampft und der so erhaltene Rückstand unter vermindertem Druck von 1,A Pa und einer Badtemperatur von 80 °C für 1,5 Stunde getrocknet.
Es werden 2.56 g einer öliger Flüssigkeit von Di(1-Ethyl-3-methylimidazolium)pentafluoroethylphosphonat erhalten, Die Ausbeute beträgt 85,0 %, bezogen auf die eingesetzte Pentafluoroethylphosponsäure.
Di(1-Ethyl-3-methylimidazolium)pentafluoroethylphosphonat wird mittels ¹⁹F, ³¹P und ¹H-NMR-Spektroskopie charakterisiert:

¹⁹F NMR spectrum, ppm :
(Lösungsmittel: Acetonitril-D₃ ; Referenz: CCl₃F);
-79,68 s (CF₃) ; -123,22 d (CF₂) ; J²P,F = 57.9 Hz.

¹H NMR spectrum, ppm:
(Lösungsmittel: Acetonitril-D₃ ; Referenz: TMS):
1,38 t (3H, CH₃) ; 3,94 s (3H, CH₃) ; 4,29 q (2H, CH₂) ; 7.70 s (1 H) ; 7.75 s (1H) ; 10.82 s (1 H) ; J³_{H,H} = 7.2 Hz.

³¹P NMR spectrum, ppm:
(Lösungsmittel: Acetonitril-D₃ : Referenz: 85 % H₃PO₄):
-1,28 t ; J²_{p,F} =57.4 Hz.

### Beispiel 8:

Eine Lösung 2,4 g (12,0 mmol) von gemäß Beispiel 4 hergestellten Pentafluorethylphosphonsäure in 13 cm³ Wasser wird mit 14,86 g von ca. 40-Gew.% wässrigen Tetrabutylphosphoniumhydroxid durch langsame Zugabe bei Raumtemperatur unter Rühren neutralisiert (pH = 7). Das Wasser wird unter vermindertem Druck abgedampft und der so erhaltene Rückstand unter vermindertem Druck von 1,4 Pa und einer Badtemperatur von 70 °C für 2 Stunde getrocknet.
Es werden 7.95 g einer hochviskosen Flüssigkeit erhalten die langsam als weißer Feststoff Bis(tetrabutylphosphonium)pentafluoroethyl-phosphonat kristallisiert. Die Ausbeute beträgt 92,4 % , bezogen auf die eingesetzte Pentafluorethylphosphonsäure. Der Schmelzpunkt beträgt 76-79° C.
Bis(tetrabutylphosphonium)pentafluoroethylphosphonat, [(C₄H₉)₄P⁺]₂ C₂F₅P(O)O₂²⁻ , wird mittels ¹⁹F, ³¹p und ¹H-NMR-Spektroskopie charakterisiert:

¹⁹F NMR spectrum, ppm :
(Lösungsmittel: Acetonitril-D₃ ; Referenz: CCl₃F):
-79,39 s (CF₃) ; -121,98 d (CF₂) ; J²_{P,F} = 54,2 Hz.

¹H NMR spectrum, ppm:
(Lösungsmittel: Acetonitril-D₃ ; Referenz: TMS):
0,93 t (12H, 4CH₃) ; 1,45 m (16H, 8CH₂) ; 2,37 m (8H, 4CH₂) J³_{H.H} = 7.1 Hz.

³¹P NMR spectrum, ppm:
(Lösungsmittel: Acetonitril-D₃ ; Referenz: 85 % H₃PO₄):
-1.84 t (1P) ; 32,73 m (2P); J²_{P,F} =54.6 Hz.

## Patentansprüche

1. Verfahren zur Herstellung von Monohydro-Perfluoralkanen oder Monohydro-Perfluoralkanen und Bis(perfluoralkyl)phosphinaten oder Perfluoralkylphosphonaten umfassend zumindest die Behandlung wenigstens eines Perfluoralkylphosphorans mit wenigstens einem Erdalkalimetallhydroxid, einer metallorganischen Verbindung oder einer organischen Base und gegebenenfalls einer Säure in Wasser und/oder einem oder mehreren organischen Lösungsmitteln als Reaktionsmedium.

2. Verfahren gemäß Anspruch 1, **dadurch gekennzeichnet, dass** das Perfluoralkylphosphoran mit wenigstens einem Erdalkalimetallhydroxid in einem geeigneten Lösungsmittel behandelt wird und anschließend die neben den Monohydro-Perfluoralkanen entstehenden Bis(perfluoralkyl)phosphinate und Perfluora(kylphosphonate direkt oder nach Isolierung mit einer Säure, vorzugsweise Schwefelsäure, in die entsprechenden Bis(perfluoralkyl)phosphinsäuren und Perfluoralkylphosphonsäuren überführt werden.

3. Verfahren gemäß Anspruch 2, **dadurch gekennzeichnet, dass** die neben den Monohydro-Perfluoralkanen entstehenden Bis(perfluoralkyl)phosphinate und Perfluoralkylphosphonate direkt oder nach Isolierung durch Umsalzung und anschließender Behandlung mit einer Säure, vorzugsweise Schwefelsäure, in die entsprechenden Bis(perfluoralkyl)phosphinsäuren und Perfluorafkylphosphonsäuren überführt werden und durch anschließende Neutralisation, vorzugsweise mit organischen Basen, die Salze gewonnen werden.

4. Verfahren gemäß einem oder mehreren der Ansprüche 1 bis 3, **dadurch gekennzeichnet, dass** als Perfluoralkylphosphoran eine Verbindung der allgemeinen Formel 1
(CₙF₂ₙ₊₁)ₘPF₅₋ₘ
eingesetzt wird, worin 1 ≤ n≤ 8, vorzugsweise 1≤ n≤ 4, und m jeweils gleich 1, 2 oder 3 bedeutet.

5. Verfahren gemäß einem oder mehreren der Ansprüche 1 bis 4, **dadurch gekennzeichnet, dass** das Pertluoralkylphosphoran ausgewählt ist aus der Gruppe bestehend aus Difluortris(pentafluorethyl)phosphoran, Difluortris(n-nonafluorbutyl)phosphoran, Difluortris(n-heptafluorpropyl)phosphoran und Trifluorbis(n- - nonafluorbutyl)phosphoran.

6. Verfahren gemäß einem oder mehreren der Ansprüche 1 bis 5, **dadurch gekennzeichnet, dass** das Verfahren bei einer Prozesstemperatur von - 10°C bis 100°C und einem moläquivalenten Verhältnis von Perfluoralkylphosphoran zu Erdalkalimetallhydroxid, metallorganischer Verbindung oder organischer Base von 1 : 3 durchgeführt wird.

7. Verfahren gemäß einem oder mehreren der Ansprüche 1 bis 6, **dadurch gekennzeichnet, dass** das Verfahren bei einer Prozesstemperatur von 50°C bis 150°C und einem moläquivalenten Verhältnis von Perfluoralkylphosphoran zu Erdalkalimetallhydroxid, metallorganischer Verbindung oder organischer Base von 1 : 4 durchgeführt wird.

8. Verfahren gemäß einem oder mehreren der Ansprüche 1 bis 7, **dadurch gekennzeichnet, dass** das Verfahren bei einer Prozesstemperatur von 100°C bis 250°C und einem moläquivalenten Verhältnis von Perflüoralkylphosphoran zu Erdalkalimetallhydroxid, metallorganischer Verbindung oder organischer Base von 1 : 5 durchgeführt wird.

9. Verfahren gemäß einem oder mehreren der Ansprüche 1 bis 8, **dadurch gekennzeichnet, dass** das Erdalkalimetallhydroxid ausgewählt ist aus der Gruppe bestehend aus Bariumhydroxid, Bariumhydroxidoctahydrat und Calciumhydroxid.

10. Verfahren gemäß einem oder mehreren der Ansprüche 1 bis 9, **dadurch gekennzeichnet, dass** die metallorganischen Verbindungen ausgewählt werden aus der Gruppe bestehend aus Metallalkyloxiden, vorzugsweise AlkalimetaHalkoxiden, Metallaryloxiden, Metallalltylthiooxiden, Metallarylthiooxiden, Alkylmetallen, Arylmetallen und Grignard-Reagenzien.

11. Verfahren gemäß einem oder mehreren der Ansprüche 1 bis 10, **dadurch gekennzeichnet, dass** die organische(n) Base(n) ausgewählt ist/sind aus der Gruppe bestehend aus Alkylammoniumhydroxiden, Arylammoniumhydroxiden, Alkylarylammoniumhydroxiden, Alkylphosphoniumhydroxiden, Arylphosphoniumhydroxiden, AJkylarylphosphoniumhydroxiden Alkylaminen, Arylaminen, Alkylarylaminen, Alkylphosphine, Arylphosphine und Alkylarylphosphine.

12. Verfahren gemäß einem oder mehreren der Ansprüche 1 bis 11, **dadurch gekennzeichnet, dass** das Reaktionsmedium Wasser im Gemisch mit einem oder mehreren organischen Lösungsmitteln ist.

13. Verfahren gemäß einem oder mehreren der Ansprüche 1 bis 11, **dadurch gekennzeichnet, dass** als Reaktionsmedium ein oder mehrere organische Lösungsmittel zum Einsatz kommen.

14. Verfahren gemäß Anspruch 12 oder 13, **dadurch gekennzeichnet, dass** das organische Lösungsmittel ausgewählt ist aus der Gruppe bestehend aus Alkoholen, Ethern, Acylamiden, Sulfoxiden, Sulfonen, Nitrilen und Kohlenwasserstoffen.

15. Verfahren gemäß Anspruch 14, **dadurch gekennzeichnet, dass** der Alkohol ein bis vier Kohlenstoffatome im Alkylteil aufweist, vorzugsweise ausgewählt ist aus der Gruppe bestehend aus Methanol, Ethanol, isoPropanol und Mischungen aus wenigstens zwei dieser Alkohole.

## Claims

1. Process for the preparation of monohydroperfluoroalkanes or monohydroperfluoroalkanes and bis(perfluoroalkyl)phosphinates or perfluoroalkylphosphonates, comprising at least the treatment of at least one perfluoroalkylphosphorane with at least one alkaline-earth metal hydroxide, an organometallic compound or an organic base and optionally an acid in water and/or one or more organic solvents as reaction medium.

2. Process according to Claim 1, **characterised in that** the perfluoroalkylphosphorane is treated with at least one alkaline-earth metal hydroxide in a suitable solvent, and the bis(perfluoroalkyl)phosphinates and perfluoroalkylphosphonates formed in addition to the monohydroperfluoroalkanes are subsequently converted directly or after isolation using an acid, preferably sulfuric acid, into the corresponding bis(perfluoroalkyl)-phosphinic acids and perfluoroalkylphosphonic acids.

3. Process according to Claim 2, **characterised in that** the bis(perfluoroalkyl)phosphinates and perfluoroalkylphosphonates formed in addition to the monohydroperfluoroalkanes are converted directly or after isolation by salt interchange and subsequent treatment with an acid, preferably sulfuric acid, into the corresponding bis(perfluoroalkyl)phosphinic acids and perfluoroalkylphosphonic acids, and the salts are obtained by subsequent neutralisation, preferably using organic bases.

4. Process according to one or more of Claims 1 to 3, **characterised in that** the perfluoroalkylphosphorane employed is a compound of the general formula I
(CₙF₂ₙ₊₁)ₘPF₅₋ₘ
in which 1 ≤ n≤ 8, preferably 1 ≤ n ≤ 4, and m in each case denotes 1, 2 or 3.

5. Process according to one or more of Claims 1 to 4, **characterised in that** the perfluoroalkylphosphorane is selected from the group consisting of difluorotris(pentafluoroethyl)phosphorane, difluorotris(n-nonafluorobutyl)phosphorane, difluorotris(n-heptafluoropropyl)phosphorane and trifluorobis(n-nonafluorobutyl)phosphorane.

6. Process according to one or more of Claims 1 to 5, **characterised in that** the process is carried out at a process temperature of -10°C to 100°C and a mole-equivalent ratio of perfluoroalkylphosphorane to alkaline-earth metal hydroxide, organometallic compound or organic base of 1:3.

7. Process according to one or more of Claims 1 to 6, **characterised in that** the process is carried out at a process temperature of 50°C to 150°C and a mole-equivalent ratio of perfluoroalkylphosphorane to alkaline-earth metal hydroxide, organometallic compound or organic base of 1:4.

8. Process according to one or more of Claims 1 to 7, **characterised in that** the process is carried out at a process temperature of 100°C to 250°C and a mole-equivalent ratio of perfluoroalkylphosphorane to alkaline-earth metal hydroxide, organometallic compound or organic base of 1:5.

9. Process according to one or more of Claims 1 to 8, **characterised in that** the alkaline-earth metal hydroxide is selected from the group consisting of barium hydroxide, barium hydroxide octahydrate and calcium hydroxide.

10. Process according to one or more of Claims 1 to 9, **characterised in that** the organometallic compounds are selected from the group consisting of metal alkoxides, preferably alkali metal alkoxides, metal aryloxides, metal alkylthiooxides, metal arylthiooxides, alkylmetal compounds, arylmetal compounds and Grignard reagents.

11. Process according to one or more of Claims 1 to 10, **characterised in that** the organic base(s) is (are) selected from the group consisting of alkylammonium hydroxides, arylammonium hydroxides, alkylarylammonium hydroxides, alkylphosphonium hydroxides, arylphosphonium hydroxides, alkylarylphosphonium hydroxides, alkylamines, arylamines, alkylarylamines, alkylphosphines, arylphosphines and alkylarylphosphines.

12. Process according to one or more of Claims 1 to 11, **characterised in that** the reaction medium is water in a mixture with one or more organic solvents.

13. Process according to one or more of Claims 1 to 11, **characterised in that** the reaction medium used is one or more organic solvents.

14. Process according to Claim 12 or 13, **characterised in that** the organic solvent is selected from the group consisting of alcohols, ethers, acylamides, sulfoxides, sulfones, nitriles and hydrocarbons.

15. Process according to Claim 14, **characterised in that** the alcohol has one to four carbon atoms in the alkyl moiety and is preferably selected from the group consisting of methanol, ethanol, isopropanol and mixtures of at least two of these alcohols.

## Revendications

1. Procédé de préparation de monohydroperfluoroalcanes ou de monohydroperfluoroalcanes et de bis(perfluoroalkyl)phosphinates ou de perfluoroalkylphosphonates, comprenant au moins le traitement d'au moins un perfluoroalkylphosphorane par au moins un hydroxyde de métal alcalino-terreux, un composé organométallique ou une base organique et éventuellement un acide dans l'eau et/ou un ou plusieurs solvants organiques comme milieu réactionnel.

2. Procédé selon la revendication 1, **caractérisé en ce que** le perfluoroalkylphosphorane est traité par au moins un hydroxyde de métal alcalino-terreux dans un solvant convenable, et les bis(perfluoroalkyl)-phosphinates et perfluoroalkylphosphonates formés en plus des monohydroperfluoroalcanes sont ensuite convertis directement ou après isolement à l'aide d'un acide, préférablement l'acide sulfurique, en acides bis(perfluoroalkyl)phosphiniques et acides perfluoroalkylphosphoniques correspondants.

3. Procédé selon la revendication 2, **caractérisé en ce que** les bis(perfluoroalkyl)phosphinates et les perfluoroalkylphosphonates formés en plus des monohydroperfluoroalcanes sont convertis directement ou après isolement par échange de sel et traitement consécutif par un acide, préférablement l'acide sulfurique, en acides bis(perfluoroalkyl)-phosphiniques et acides perfluoroalkylphosphoniques correspondants, et les sels sont obtenus par neutralisation consécutive, préférablement à l'aide de bases organiques.

4. Procédé selon l'une ou plusieurs des revendications 1 à 3, **caractérisé en ce que** le perfluoroalkylphosphorane employé est un composé de formule générale I
(CₙF₂ₙ₊₁)mPF₅₋ₘ
dans laquelle 1 ≤ n ≤ 8, préférablement 1 ≤ n ≤ 4, et m désigne dans chaque cas 1, 2 ou 3.

5. Procédé selon l'une ou plusieurs des revendications 1 à 4, **caractérisé en ce que** le perfluoroalkylphosphorane est choisi parmi le groupe constitué par le difluorotris(pentafluoroéthyl)phosphorane, le difluorotris-(n-nonafluorobutyl)phosphorane, le difluorotris(n-heptafluoropropyl)-phosphorane et le trifluorobis(n-nonafluorobutyl)phosphorane.

6. Procédé selon l'une ou plusieurs des revendications 1 à 5, **caractérisé en ce que** le procédé est réalisé à une température de procédé allant de -10°C à 100°C et à un rapport d'équivalent molaire de perfluoroalkylphosphorane à l'hydroxyde de métal alcalino-terreux, au composé organométallique ou à la base organique de 1:3.

7. Procédé selon l'une ou plusieurs des revendications 1 à 6, **caractérisé en ce que** le procédé est réalisé à une température de procédé allant de 50°C à 150°C et à un rapport d'équivalent molaire de perfluoroalkylphosphorane à l'hydroxyde de métal alcalino-terreux, au composé organométallique ou à la base organique de 1:4.

8. Procédé selon l'une ou plusieurs des revendications 1 à 7, **caractérisé en ce que** le procédé est réalisé à une température de procédé allant de 100°C à 250°C et à un rapport d'équivalent molaire de perfluoroalkylphosphorane à l'hydroxyde de métal alcalino-terreux, au composé organométallique ou à la base organique de 1:5.

9. Procédé selon l'une ou plusieurs des revendications 1 à 8, **caractérisé en ce que** l'hydroxyde de métal alcalino-terreux est choisi parmi le groupe constitué par l'hydroxyde de baryum, l'hydroxyde de baryum octahydraté et l'hydroxyde de calcium.

10. Procédé selon l'une ou plusieurs des revendications 1 à 9, **caractérisé en ce que** les composés organométalliques sont choisis parmi le groupe constitué par les alkylates métalliques, préférablement les alkoxydes de métal alcalin, les arylates métalliques, les alkylthiooxydes métalliques, les arylthiooxydes métalliques, les composés d'alkylmétal, les composés d'arylmétal et les réactifs de Grignard.

11. Procédé selon l'une ou plusieurs des revendications 1 à 10, **caractérisé en ce que** la ou les bases organiques sont choisies parmi le groupe constitué par les hydroxydes d'alkylammonium, les hydroxydes d'arylammonium, les hydroxydes d'alkylarylammonium, les hydroxydes d'alkylphosphonium, les hydroxydes d'arylphosphonium, les hydroxydes d'alkylarylphosphonium, les alkylamines, les arylamines, les alkylarylamines, les alkylphosphines, les arylphosphines et les alkylarylphosphines.

12. Procédé selon l'une ou plusieurs des revendications 1 à 11, **caractérisé en ce que** le milieu réactionnel est de l'eau en mélange avec un ou plusieurs solvants organiques.

13. Procédé selon l'une ou plusieurs des revendications 1 à 11, **caractérisé en ce que** le milieu réactionnel utilisé est constitué d'un ou plusieurs solvants organiques.

14. Procédé selon la revendication 12 ou 13, **caractérisé en ce que** le solvant organique est choisi parmi le groupe constitué par les alcools, les éthers, les acylamides, les sulfoxydes, les sulfones, les nitriles et les hydrocarbures.

15. Procédé selon la revendication 14, **caractérisé en ce que** l'alcool comporte de un à quatre atomes de carbone dans le motif alkyle et est choisi de préférence parmi le groupe constitué par le méthanol, l'éthanol, l'isopropanol et des mélanges d'au moins deux parmi ces alcools.
